# FASCICULE DE BREVET EUROPEEN

(11) **EP 1 103 233 B1**
(45) Date de publication et mention de la délivrance du brevet: **10.08.2005**
(21) Numéro de dépôt: 00403309.8
(22) Date de dépôt: 27.11.2000
(51) Int. Cl.: A61F 2/01

(54) **Procédé de fabrication d'un filtre sanguin monobloc**
Herstellungsverfahren eines Monoblockblutfilters
Monobloc blood filter production method

(30) Priorité: 26.11.1999 FR 9914900
(43) Date de publication de la demande: 30.05.2001
(73) Titulaire: B. Braun Medical SAS, 92100 Boulogne Billancourt (FR)
(72) Inventeur: Guy, M. Nadal, 86000 Poitiers (FR)
(74) Mandataire: Lerner, François

(56) Documents cités:
- EP-A- 0 759 287
- EP-A- 0 815 803
- WO-A-99/23976
- WO-A-99/25252
- US-A- 5 938 683

## Description

L'invention concerne un procédé d'obtention d'un filtre sanguin adapté pour être disposé à l'intérieur d'un vaisseau d'un corps humain ou animal, ainsi que le filtre en lui-même.

Il existe déjà des filtres sanguins.

Pour fabriquer ces filtres, on prévoit typiquement de créer, pour chaque filtre, des pattes allongées et (au moins) une tête qui réunit ces pattes entre elles localement, avec une disposition des pattes telle qu'elles s'étendent autour d'un axe principal et avec une capacité donnée à ces pattes d'être mobiles radialement par rapport audit axe principal et à la tête, entre une première position radialement resserrée et une seconde position radialement écartée dans laquelle les pattes sont, sur une partie au moins de leur longueur, plus écartées de l'axe que dans la première position.

Des exemples peuvent être trouvés dans FR-A-2 573 646 (auquel correspond US-A-4 688 553) ou dans FR-A-2 694 491 (auquel correspond US-A-5 344 427), ou dans WO-A-99 25 252.

Dans cet art antérieur, il est ainsi connu, pour fabriquer un filtre,
a) d'utiliser une paroi comprenant un matériau biocompatible, et
b) pour définir lesdites pattes, de créer dans cette paroi une série de tronçons de matière, allongés, essentiellement parallèles entre eux, ces tronçons ayant une première extrémité libre et une seconde extrémité opposée axialement

L'invention a pour objet d'améliorer les conditions de fabrication d'un tel filtre sanguin.

L'objectif est en particulier le suivant :
- réduire les opérations d'assemblage des parties du filtre entre elles,
- réduire les risques mécaniques consécutifs à une augmentation locale des contraintes lors de la fabrication,
- améliorer la cohésion entre les parties du filtre,
- définir une réduction potentielle des coûts de fabrication,
- profiter des développements techniques intervenus dans le domaine général des "dispositifs vasculaires" depuis quelques années, en cherchant d'éventuelles synergies qui pourraient exister entre eux et les filtres sanguins.

Pour tendre vers cet objectif, une caractéristique importante de l'invention prévoit que le procédé de fabrication du filtre comprenne les étapes suivantes :
c) dans certains au moins des tronçons de matière matérialisés lors de l'étape b) précitée, des bandes de matière adjacentes, essentiellement parallèles entre elles, comprenant au moins une première bande et une seconde bande séparées par une fente, sont créées, les bandes s'étendant essentiellement parallèlement à l'axe et les fentes s'interrompant à distance de la première extrémité libre des tronçons concernés, de sorte que les premières et les secondes bandes sont monoblocs en étant liées entre elles à cette extrémité libre par une zone de matière de la paroi, tandis que les secondes bandes sont libres vis-à-vis des premières bandes, le long des fentes (et en particulier vers la seconde extrémité desdits tronçons),
d) et les premières bandes sont reliées entre elles vers cette seconde extrémité.

Bien que la limite des contraintes mécaniques soit un objectif affiché, l'invention prévoit de préférence une étape supplémentaire de décalage angulaire des secondes bandes par rapport aux premières, de sorte que dans la seconde position, les premières bandes sont inclinées par rapport à l'axe tandis que les secondes bandes s'étendent sensiblement parallèlement à cet axe.

Il est à noter que ce "décalage angulaire" est typiquement de l'ordre de 30° à 40°, ce qui est bien moins que le repliage presque en épingle à cheveux de zones du(des) fil(s) de fabrication du filtre dans US-A-4 688 553 ou encore dans US-A-5 344 427, voire dans US-A-5 383 887.

En outre, on évite la soudure comme dans FR-A-2 764 503.

A la place d'une action mécanique, un traitement thermique associé à l'utilisation d'un matériau à mémoire de forme (tel qu'un alliage superélastique NiTi) pourrait être utilisé, y compris pour faire bouger le filtre entre ses premier et second états.

Pour favoriser la qualité de fabrication, la fiabilité du filtre dans le temps, ainsi que les coûts de cette fabrication, on conseille par ailleurs, lors de l'étape a) précitée, d'utiliser une paroi tubulaire et, lors de l'étape b), après création des fentes, de relier entre elles les premières bandes en rapportant une pièce de liaison à laquelle on fixera les premières bandes, à la seconde extrémité, ces bandes étant alors régulièrement réparties angulairement autour de l'axe.

Concernant maintenant plus particulièrement l'aspect filtre en lui-même, une caractéristique importante de l'invention peut être énoncée comme suit, toujours pour satisfaire à l'objectif global énoncé en début de description :
- les tronçons "découpés" dans la paroi initialement utilisée se présentent individuellement eux-mêmes en forme de plaque, à section non circulaire, et comprennent au moins deux bandes séparées par une fente qui s'interrompt à distance de la première extrémité, de sorte que les premières et secondes bandes y sont réunies de façon monobloc en définissant les pattes, pour les premières bandes qui sont réunies ensemble, et les appendices de centrage, pour les secondes bandes.

Une description plus détaillée de l'invention va maintenant être donnée en référence aux dessins d'accompagnement dans lesquels :
- la figure 1 montre schématiquement une machine de découpe laser, à mandrin, adaptée pour la découpe du tube de la figure 2 (vue agrandie),
- la figure 3 montre schématiquement le tube de la figure 2 une fois découpé,
- la figure 4 montre en vue partielle un filtre conforme à l'invention dans son état radialement déployé,
- la figure 5 montre à plat une possibilité d'obtention d'un filtre conforme à l'invention, à partir d'une plaque plane telle qu'illustrée sur la figure 6,
- les figures 7 et 8 montrent schématiquement deux alternatives de maintien en position arrondie de la tête du filtre de la figure 5, après enroulement,
- les figures 9, 10 et 11 montrent schématiquement trois autres possibilités de réalisation de pattes et appendices pour le filtre,
- la figure 12 schématise la découpe de la forme de la figure 9 à partir d'une fine paroi tubulaire,
- et la figure 13 montre une paroi tubulaire évasée.

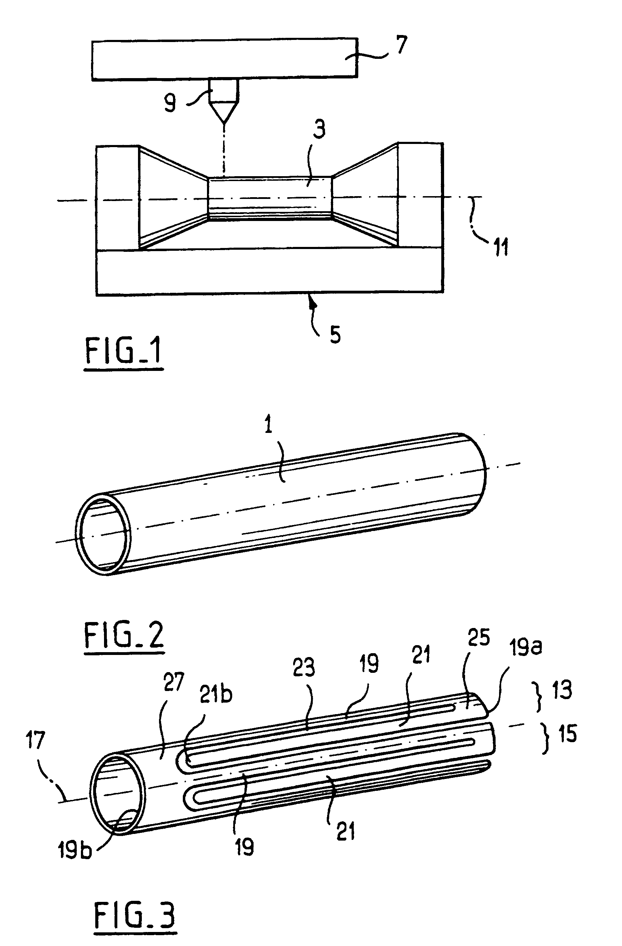

Sur la figure 1, on a schématisé la création d'un filtre conforme à l'invention, par découpe laser, comme dans l'hypothèse où l'on partirait non pas d'une plaque plane, mais d'une plaque déjà cintrée, ou d'un tube, que l'on appellera "paroi".

Une technique de découpe laser est déjà divulguée dans EP-B-0 714 641 tel que délivré (colonne 9, ligne 24 ; colonne 10, ligne 9).

Brièvement, une paroi fine tubulaire de petit diamètre, tel que le tube cylindrique de section constante 1 de la figure 2, qui peut être en acier inoxydable, est disposée autour d'un mandrin rotatif 3 d'une machine à commande numérique 5 équipée d'un bras 7 pourvu d'un laser 9, tel qu'un laser au Nd. Le tube 1 est disposé autour du mandrin 3, puis mis en rotation et déplacé de façon longitudinale (suivant l'axe 11) par rapport au laser 9, lequel est également commandé par la machine 5. Le laser découpe le tube 1 et dessine ainsi le motif voulu, correspondant en l'espèce à l'illustration schématique de la figure 3.

A noter que l'opération de "découpe" de la paroi 1 peut être réalisée de manière différente, par exemple par gravure (couramment dénommée "etching") ou par découpe avec un fin "couteau",ou encore par un laser CO₂ (voir US-A-5 421955, colonne 6, ligne 31 ; colonne 7, ligne 32).

Si on a recourt à une gravure chimique, la technique est rappelée notamment dans EP-A-0 709 767 (Colonne 2, ligne 38 ; colonne 3, ligne 28).

Brièvement, on rappellera que le dessin de la forme à découper est imprimé sur un ou deux films transparents que l'on place sur la fine paroi (ou feuille) à découper, avec une couche photorésistive. La paroi ainsi revêtue est illuminée de sorte que les parties photorésistives qui reçoivent la lumière (parties en creux de la forme définitive à obtenir) changent de propriétés. La paroi (en l'espèce métallique) est ensuite placée dans un acide qui détruit ces parties photorésistives qui ont changé de propriétés, après quoi la paroi est placée dans une solution de gravure qui détache toute la matière sur laquelle il n'y a plus cette partie photorésistive. Ensuite, les parties restantes de la paroi d'origine sont placées dans une solution de rinçage qui retire la couche photorésistive restante, permettant ainsi d'obtenir la forme souhaitée, telle que la forme de la figure 3.

Sur cette figure, on constatera que le "dessin" du filtre comprend une série de tronçons de matière tels que 13 et 15 globalement parallèles entre eux et parallèles à une direction longitudinale 17 appelée "axe principal du filtre" (correspondant à l'axe 11 du tube).

La répartition des tronçons allongés, tels que 13, 15, est régulière, angulairement, autour de l'axe 17.

Dans l'exemple, ce qui vaut donc pour un tronçon allongé, tel que 13, vaut pour les autres tronçons.

Ainsi, le tronçon 13 comprend deux bandes de matière 19, 21, séparées par une fente 23 globalement parallèle à l'axe 17. La fente 23 s'interrompt à distance de l'extrémité libre 19a de la première bande 19 et est par contre "débouchante" à l'extrémité opposée, en 21b, de sorte que les deux bandes 19, 21, sont réunies uniquement par une zone de matière 25 du côté de l'extrémité 19a, tandis qu'elles sont libres de mouvement l'une par rapport à l'autre le long de la fente 23, y compris à l'extrémité 21b qui constitue donc une extrémité libre pour la seconde bande 21.

Si l'on référencie 19b l'extrémité proximale du filtre opposée à son extrémité distale 19a, on notera également que toutes les "premières bandes", telles que 19, sont réunies entre elles par un moyen de liaison qui, en l'espèce, est constitué par un bandeau 27 de matière non découpée de la paroi, s'étendant transversalement (en l'espèce, perpendiculairement) par rapport à la direction générale 17 d'allongement des tronçons et des bandes et qui dans l'exemple, réunit de façon monobloc toutes les premières bandes (telles que 19) à proximité immédiate de l'extrémité proximale 19b.

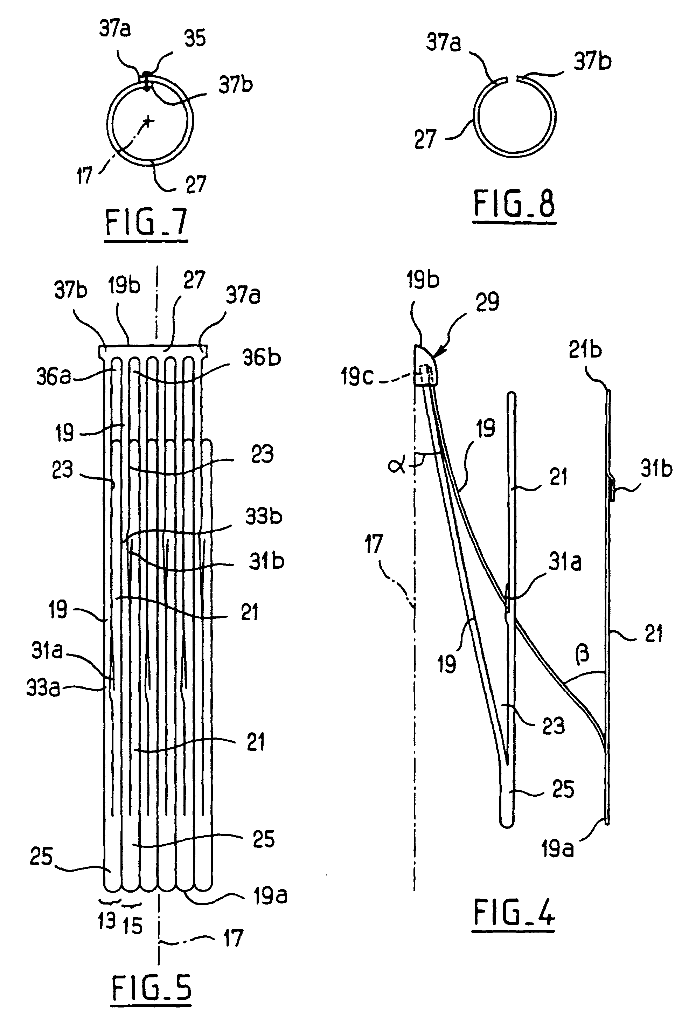

Sur la figure 4, une représentation plus conforme à la réalité d'un filtre sanguin a été représentée. Toutefois, seuls certains des tronçons découpés de ce filtre ont été figurés, pour éviter de surcharger la figure.

On notera également qu'autant sur la figure 3, le filtre peut être considéré dans son état "radialement resserré" (c'est-à-dire, tel qu'il se trouve à l'intérieur d'un dispositif introducteur), autant sur la figure 4, le filtre est dans son état "radialement déployé", c'est-à-dire comme il se trouve lorsqu'il est centré dans le vaisseau, remplissant sa fonction de filtration des thrombus.

En particulier sur la figure 4, on distingue que les premières bandes de matière telles que 19 définissent, vis-à-vis du filtre et dans cette position, des pattes inclinées par rapport à l'axe 17, tandis que les "secondes bandes" 21 définissent alors des stabilisateurs, ou appendices de centrage.

L'angle moyen d'inclinaison α, dans cette position radialement déployée, des pattes telles que 19 par rapport à l'axe 17, peut être de l'ordre de 30° à 40°, cet angle étant identique à l'angle β de décalage angulaire entre les pattes et les stabilisateurs (lesquels doivent en effet occuper une position parallèle à l'axe 17, suivant une génératrice de cylindre de section constante.

On notera que le sommet de l'angle α est situé à l'extrémité proximale 19b du filtre (couramment dénommé "tête du filtre"), le cône de filtration définit par les pattes s'ouvrant jusqu'à l'extrémité 19a où les pattes sont le plus écartées les unes des autres, dans l'état radialement déployé de la figure 4.

Parmi les matières (ou revêtements) de la paroi 1 d'origine, on peut en particulier citer : l'acier inoxydable, le "Nitinol"® qui est un alliage superélastique à mémoire de forme thermique, ou encore le titane, voire un matériau plastique (polymère thermoplastique). Ainsi, l'état de la figure 4 peut être obtenu par élasticité naturelle des bandes 19, 21, ou par variation de température si un alliage à mémoire de forme thermique a été utilisé et si la forme des figures 3 et 4 a été "enregistrée" par cette matière, selon une technique bien connue.

A noter que deux différences structurelles existent entre les illustrations des figures 3 et 4, puisque sur la figure 4 la réunion des premières bandes telles que 19, à proximité de l'extrémité 19b, est réalisée par une pièce rapportée 29 et que par ailleurs, des crochets tels que 31a, 31b, ont été intégrés aux appendices de centrage tels que 21.

Concernant la pièce rapportée 29, il s'agit d'une calotte au moins partiellement creuse à l'intérieur de laquelle les deux pattes 19 illustrées sont soudées. Dans ce cas, les pattes 19, avant d'être réunies par la calotte 29, sont indépendantes les unes des autres, c'est-à-dire que chaque fente 23 débouche vers l'extrémité proximale 19b du tube illustré sur la figure 3. Ces pattes indépendantes qui présentent donc alors une extrémité libre 19c, sont rapprochées les unes des autres à cette extrémité et fixées à la calotte 29 qui constitue donc la tête de réunion des pattes.

Concernant la formation des crochets intégrés tels que 31a, 31b, on la comprendra à partir de la figure 5 qui montre une alternative (a priori moins favorable) de fabrication du filtre de la figure 4, à partir non pas d'un tube, mais d'une plaque plane, comme la plaque 30 de la figure 6.

Sur la figure 5, cette paroi 30 est déjà "découpée", c'est-à-dire notamment que les fentes de matérialisation des bandes en forme de plaque (donc à section non circulaire) définissant respectivement les pattes (ou bras) du filtre et les appendices de centrage ont déjà été créées.

A la différence de la figure 4 où l'on a donc utilisé une tête rapportée ogivale 29, on constate sur la figure 5 que, partant de la bande supérieure de jonction 27 transversale à l'axe longitudinal 17 du filtre, les tronçons parallèles de découpe, tels que ceux repérés une nouvelle fois 13, 15, comprennent une première bande 19, amincie en 33a, 33b, et qui se prolonge à l'extrémité axiale 19a (opposée à la bande 27) par une seconde bande telle que 21 liée à la première bande par la zone de liaison en épingle à cheveux telle que 25. A hauteur des zones 33a, 33b, les secondes bandes concernées sont elles-mêmes amincies de manière à dessiner là le crochet intégré tel que 31a, 31b.

En direction de l'extrémité proximale 19b, les secondes bandes 21 s'interrompent à distance du bandeau, de sorte que les secondes bandes "retour" sont plus courtes que les premières bandes "aller". Ainsi, les espaces vides tels que 36a, 36b, sont matérialisés.

Une fois cette forme découpée, les premières bandes 19 sont déformées angulairement par rapport au bandeau 27, tandis que les secondes bandes 21 sont maintenues sensiblement parallèles à l'axe 17.

Avant ou après, l'ensemble est roulé sur lui-même autour de l'axe de révolution 17.

On obtient ainsi un filtre sanguin avec des pattes allongées présentant la forme d'une corolle sensiblement tronconique, avec une ouverture axiale dans sa tête étroite 27 et une extrémité libre 19a où, dans cet état radialement déployé, le filtre présente son plus grand diamètre.

Une soudure 35 peut maintenir le filtre enroulé, à l'endroit de la tête 27, où les deux extrémités opposées 37a, 37b, de la plaque peuvent se chevaucher, comme illustré sur la figure 7. En alternative, la figure 8 montre que ces deux mêmes extrémités peuvent se faire face, sans nécessiter de moyens de fixation entre elles.

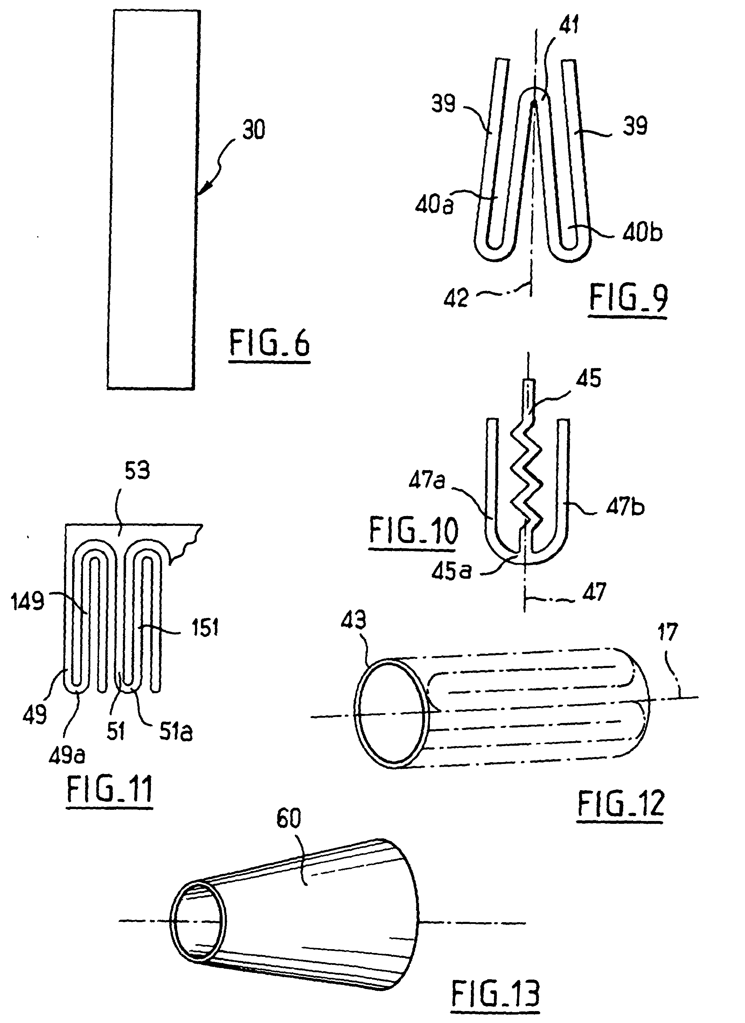

Sur les figures 9, 10 et 11, on a figuré de façon très schématique certaines parmi d'autres formes possibles des pattes et appendices de centrage du filtre.

Ainsi, sur la figure 9, on voit une forme en "W" comprenant deux bandes latérales 39 définissant les pattes, liées chacune à un appendice central 41 en "V" inversé. Deux fentes 40a, 40b définissant un "V" inversé, donc essentiellement parallèles à l'axe 42, sont matérialisées.

De telles formes peuvent être obtenues par exemple à partir du tube 43 de la figure 12, dans lequel on a découpé les bandes telles que figurées, les bandes une fois découpées étant ensuite légèrement déformées pour parvenir à la forme finale souhaitée.

Sur la figure 10, on voit une patte centrale en zigzag (donc essentiellement parallèle à l'axe 47), 45, reliée continûment à l'extrémité 45a à deux appendices latéraux 47a, 47b.

Sur la figure 11, on voit deux pattes 49, 51, réunies entre elles à une extrémité par un bandeau 53 et prolongées, à l'extrémité opposée (respectivement 49a et 51a) par un appendice, respectivement 149 et 151, en "V" inversé.

Les formes peuvent ainsi être diverses. L'important est de dessiner une ou plusieurs patte(s) et appendice(s) de centrage issus d'une paroi dans laquelle on a "découpé" la forme souhaitée, avec une(des) fente(s) globalement parallèle(s) à l'axe du filtre, lequel peut d'ailleurs être considéré comme confondu avec celui du vaisseau, une fois le filtre implanté.

A noter également qu'une paroi tubulaire évasée 60, notamment tronconique, pourrait être utilisée, en remplacement de celle de la figure 2 (voir figure 13).

## Revendications

1. Procédé pour fabriquer un filtre sanguin adapté pour être disposé dans un vaisseau, le filtre présentant un axe (17) et comprenant des pattes allongées (19), s'étendant autour de cet axe, ces pattes étant mobiles radialement entre une première position dans laquelle les pattes sont radialement écartées localement et une seconde position dans laquelle les pattes sont radialement localement plus rapprochées que dans la première position, le procédé comprenant les étapes de :
a) utiliser une paroi (1, 30) comprenant un matériau biocompatible, et
b) pour définir lesdites pattes, créer dans cette paroi une série de tronçons de matière, allongés (13, 15), essentiellement parallèles entre eux, ces tronçons ayant une première extrémité libre et une seconde extrémité opposée axialement,
**caractérisé en ce que** lors de l'étape b) :
c) dans certains au moins des tronçons, des bandes de matière adjacentes essentiellement parallèles entre elles, comprenant au moins une première bande (19, 39, 45, 49) et une seconde bande (21 ; 41, 51 ; 47a, 47b ; 149, 151) séparées par une fente (23, 40a, 40b), sont créées, les bandes s'étendant essentiellement parallèlement à l'axe et les fentes s'interrompant à distance de la première extrémité libre (19a) des tronçons concernés, de sorte que les premières et les secondes bandes sont monoblocs, en étant liées entre elles à cette extrémité libre par une zone de matière (25, 45a, 49a, 51a) de la paroi, tandis que les secondes bandes sont libres vis-à-vis des premières bandes le long desdites fentes,
d) et les premières bandes (19, 39, 45, 49) sont reliées entre elles vers cette seconde extrémité (19b).

2. Procédé selon la revendication 1, **caractérisé en ce que** lors de l'étape a), on utilise une paroi tubulaire et lors de l'étape b), après création des fentes, les premières bandes sont reliées entre elles en rapportant une pièce de liaison (29) à laquelle on fixe, à la deuxième extrémité (19b), les premières bandes (19), lesquelles sont régulièrement réparties angulairement autour de l'axe (17).

3. Procédé selon la revendication 1 ou la revendication 2, **caractérisé en ce qu'**il comprend en outre une étape de décalage angulaire des secondes bandes (21) par rapport aux premières (19), de sorte que dans la seconde position, les premières bandes sont inclinées par rapport à l'axe (17) tandis que les secondes bandes s'étendent sensiblement parallèlement à cet axe.

4. Filtre sanguin adapté pour être disposé à l'intérieur d'un vaisseau, le filtre présentant un axe (17) et comprenant une série de tronçons (13, 15) de matière, allongés, définissant localement des pattes, lesquelles sont angulairement réparties autour de l'axe et présentent une première extrémité '(19a) où elles sont liées à des appendices de centrage du filtre, ces appendices (21) s'étendant naturellement essentiellement parallèlement à l'axe, et une seconde extrémité (19b) vers laquelle elles sont réunies ensemble, de sorte qu'à la première extrémité les pattes et les appendices présentent un premier état dans lequel ils sont radialement écartés et un second état dans lequel ils sont rapprochés de l'axe,
**caractérisé en ce que** les tronçons se présentent individuellement en forme de plaque à section non circulaire, comprenant au moins deux bandes (19, 21) séparées par une fente (23, 40a, 40b) qui s'interrompt à distance de la première extrémité (19a), de sorte que les premières et secondes bandes y sont réunies de façon monobloc, en définissant les pattes, pour les premières bandes, et les appendices de centrage, pour les secondes bandes.

5. Filtre sanguin selon la revendication 4, **caractérisé en ce que** les premières bandes (19, 39, 45, 49) sont réunies entre elles par un bandeau de matière (27) monobloc avec elles, et s'étendant transversalement à l'axe (17).

## Claims

1. A method of manufacturing a blood filter suitable for being arranged in a vessel, the filter having an axis (17) and comprising elongate flaps (19) extending around this axis, these flaps being radially mobile between a first position in which the flaps are locally radially spaced apart and a second position in which the flaps are locally radially closer together than in the first position, the method comprising the steps of:
a) use of a wall (1, 30) comprising a biocompatible material, and
b) for the purpose of defining the said flaps, creating in this wall a series of elongate lengths of material (13, 15) substantially parallel to one another, these lengths having a first, free end and a second, axially opposite end,
**characterised in that**, during step b):
c) in at least some of the lengths, adjacent bands of material are created, which bands are substantially parallel to one another and comprise at least one first band (19, 39, 45, 49) and a second band (21; 41, 51; 47a, 47b; 149, 151) separated by a slit (23, 40a, 40b), the bands extending substantially parallel to the axis and the slits stopping at a distance from the first, free end (19a) of the respective lengths, in such a way that the first and the second bands are formed from a single piece, being connected at this free end by a zone of material (25, 45a, 49a, 51a) of the wall, whereas the second bands are free relative to the first bands along the said slits,
d) and the first bands (19, 39, 45, 49) are recombined towards this second end (19b).

2. A method according to Claim 1, **characterised in that**, during step a), a tubular wall is used and, during step b), following creation of the slits, the first bands are recombined by insertion of a binding member (29) to which, at the second end (19b), the first bands (19) are attached regularly distributed about the axis (17).

3. A method according to Claim 1 or Claim 2, **characterised in that** it also comprises a step of angular displacement of the second bands (21) relative to the first bands (19) in such a way that, in the second position, the first bands are inclined relative to the axis (17) whereas the second bands extend substantially parallel to this axis.

4. A method of manufacturing a blood filter suitable for being arranged in the interior of a vessel, the filter having an axis (17) and comprising a series of elongate lengths (13, 15) of material, locally defining flaps, which flaps are angularly distributed about the axis and have a first end (19a) where they are connected to filter centring appendages, these appendages (21) naturally extending substantially parallel to the axis, and a second end (19b) towards which they are re-united in a group, in such a way that, at the first end, the flaps and the appendages present a first state in which they are radially distanced from the axis, and a second state in which they are brought close thereto,
**characterised in that** the lengths are each in the form of a sheet of non-circular cross-section, comprising at least two bands (19, 21) separated by a slit (23, 40a, 40b) which terminates at a distance from the first end (19a), in such a way that the first and second bands are there joined together so as to be of one piece, defining the flaps in the case of the first bands and the centring appendages in the case of the second bands.

5. A blood filter according to Claim 4, **characterised in that** the first bands (19, 39, 45, 49) are joined together by a band of material (27) of a piece therewith and extending transversely to the axis (17).

## Patentansprüche

1. Verfahren zur Herstellung eines Blutfilters, der geeignet ist, in einem Gefäß angeordnet zu werden, wobei der Filter eine Achse (17) aufweist und längliche Stützen (19) hat, die sich um diese Achse erstrecken, wobei diese Stützen radial zwischen einer ersten Position, in der sich die Stützen radial lokal im Abstand befinden, und einer zweiten Position, in der sich die Stützen radial lokal näher aneinander als in der ersten Position befinden, beweglich sind, wobei das Verfahren die Schritte aufweist:
a) Verwenden einer Wand (1, 30), die ein biokompatibles Material aufweist, und
b) Bilden einer Reihe von länglichen, im wesentlichen zueinander parallelen Materialabschnitten (13, 15) in dieser Wand, um die Stützen zu bestimmen, wobei diese Abschnitte ein erstes freies Ende und ein axial gegenüberliegendes zweites Ende haben,
**dadurch gekennzeichnet, daß** während des Schrittes b):
c) mindestens in einigen Abschnitten im wesentlichen zueinander parallele, nebeneinanderliegende Materialbänder gebildet sind, die mindestens ein erstes Band (19, 39, 45, 49) und ein zweites Band (21; 41, 51; 47a, 47b; 149, 151) aufweisen, die durch einen Spalt (23, 40a, 40b) getrennt sind, wobei sich die Bänder im wesentlichen parallel zu der Achse erstrecken und die Spalte im Abstand des ersten freien Endes (19a) der betreffenden Abschnitte derart unterbrochen sind, daß die ersten und zweiten Bänder einstückig sind, wobei sie untereinander an diesem freien Ende durch einen Materialbereich (25, 45a, 49a, 51a) der Wand verbunden sind, während die zweiten Bänder gegenüber den ersten Bändern entlang der Spalte frei sind,
d) und die ersten Bänder (19, 39, 45, 49) untereinander zu diesem zweiten Ende (19b) hin verbunden sind.

2. Verfahren nach Anspruch 1, **dadurch gekennzeichnet, daß** während des Schritts a) eine rohrförmige Wand verwendet wird, und während des Schritts b) nach Bildung der Spalte die ersten Bänder untereinander unter Anfügen eines Verbindungsstücks (29) verbunden werden, an welches an dem zweiten Ende (19b) die ersten Bänder (19) befestigt werden, die regelmäßig winklig um die Achse (17) verteilt sind.

3. Verfahren nach Anspruch 1 oder Anspruch 2, **dadurch gekennzeichnet, daß** es ferner einen Schritt winkligen Versetzens der zweiten Bänder (21) bezüglich der ersten (19) derart aufweist, daß die ersten Bänder in der zweiten Position bezüglich der Achse (17) geneigt sind, während sich die zweiten Bänder im wesentlichen parallel zu dieser Achse erstrecken.

4. Blutfilter, der für die Anordnung im Inneren eines Gefäßes geeignet ist, wobei der Filter eine Achse (17) aufweist und eine Reihe von länglichen Materialabschnitten (13, 15) hat, die lokal Stützen bestimmen, welche im Winkel um die Achse verteilt sind und ein erstes Ende '(19a) aufweisen, wo sie mit Zentrieransätzen des Filters verbunden sind, sich diese Ansätze (21) selbstverständlich im wesentlichen parallel zu der Achse erstrecken und ein zweites Ende (19b) aufweisen, zu dem hin sie derart zusammen verbunden sind, daß die Stützen und die Ansätze an dem ersten Ende einen ersten Zustand aufweisen, in dem sie sich radial voneinander im Abstand befinden, und einen zweiten Zustand, in dem sie sich nahe der Achse befinden,
**dadurch gekennzeichnet, daß** sich die Abschnitte einzeln in der Form einer Platte mit nicht kreisrundem Querschnitt darstellen, wobei sie mindestens zwei Bänder (19, 21) aufweisen, die durch einen Spalt (23, 40a, 40b) getrennt sind, der im Abstand von dem ersten Ende (19a) derart aufhört, daß die ersten und zweiten Bänder dort einstückig verbunden werden, wobei die Stützen für die ersten Bänder und die Zentrieransätze für die zweiten Bänder definiert werden.

5. Blutfilter nach Anspruch 4, **dadurch gekennzeichnet, daß** die ersten Bänder (19, 39, 45, 49) untereinander durch einen einstückigen Materialgurt (27), der sich quer zu der Achse (17) erstreckt, mit sich verbunden sind.
